(19) **Europäisches Patentamt**

**European Patent Office**

**Office européen des brevets**

(11) Publication number: **0 579 202 A1**

## (12) EUROPEAN PATENT APPLICATION

(21) Application number: **93111290.8**

(22) Date of filing: **14.07.93**

(51) Int. Cl.5: **C12Q 1/00**, G01N 33/52, C12Q 1/32, C12Q 1/50, C12Q 1/54, C12Q 1/60, C12Q 1/61, C12Q 1/62

(30) Priority: **15.07.92 US 914826**

(43) Date of publication of application:
**19.01.94 Bulletin 94/03**

(84) Designated Contracting States:
**CH DE FR GB LI NL**

(71) Applicant: **EASTMAN KODAK COMPANY**
**343 State Street**
**Rochester, New York 14650-2201(US)**

(72) Inventor: **Arter, Thomas Charles, c/o**
**EASTMAN KODAK COMPANY**
**Patent Legal Staff,**
**343 State Street**
**Rochester, New York 14650-2201(US)**
Inventor: **Warren, Karen Lee, c/o EASTMAN**
**KODAK COMPANY**
**Patent Legal Staff,**
**343 State Street**
**Rochester, New York 14650-2201(US)**
Inventor: **Warren, Harold Chester, c/o**
**EASTMAN KODAK COMPANY**

**Patent Legal Staff,**
**343 State Street**
**Rochester, New York 14650-2201(US)**
Inventor: **Snoke, Roy Eugene, c/o EASTMAN**
**KODAK COMPANY**
**Patent Legal Staff,**
**343 State Street**
**Rochester, New York 14650-2201(US)**
Inventor: **Schaeffer, James Robert, c/o**
**EASTMAN KODAK COMPANY**
**Patent Legal Staff,**
**343 State Street**
**Rochester, New York 14650-2201(US)**
Inventor: **Decann, Carol Anne, c/o Eastman**
**Kodak Company**
**Patent Legal Staff, 343 State Street**
**Rochester, New York, 14650-2201(US)**

(74) Representative: **Brandes, Jürgen, Dr. et al**
**Wuesthoff & Wuesthoff**
**Patent- und Rechtsanwälte**
**Schweigerstrasse 2**
**D-81541 München (DE)**

(54) **Sulfhydryl-complexing agents in clinical test elements.**

(57) A dry analytical element for the determination of an analyte is disclosed wherein the spreading layer of the element contains a reagent that eliminates reducing interferents present in the analyte sample.

EP 0 579 202 A1

FIG. 4B

A = Standard
B = 1 mm n-acetyl cysteine
C = 1 mm Copper Sulfate and 1mm n-acetyl cysteine
D = 1 mm Copper Sulfate

This invention relates generally to the field of clinical chemistry.

In testing biological fluids for the presence of certain substances (analytes), it is known how to employ chemical reactions that provide a detectable change when analyte is present in the fluid. Often, the detectable change occurs because the analyte triggers an oxidation/reduction (redox) reaction which brings about a spectral shift in one or more reagents in the test. The presence of analyte is then determined spectrophotometrically.

Some natural components of biological fluids have free sulfhydryl groups that can trigger oxidation/reduction even in the absence of analyte. If present in the sample to be tested, such components can contribute to inaccurate results. For example, sulfhydryl groups are essential in the structure and assembly of some proteins, enzymes, and complex carbohydrates. Depending on their concentration in the sample, these natural components of serum can act as reductants and simulate presence of analyte.

Components bearing free sulfhydryl groups may also appear in the serum of patients taking certain types of medication. For example, N-acetyl cysteine (NAC) is an analog of the natural amino acid L-cysteine and has a free thiol group:

$$HSCH_2CH(NHCOCH_3)COOH$$

At standard assay pH (6.5-7.5), the carboxylic group on NAC is negatively charged, making it a very effective reducing agent. NAC is used as a therapeutic in human medicine as a mucolytic or mucoregulatory pharmaceutical and as treatment for patients overdosing on acetaminophen (paracetamol). To be effective, generally high doses of NAC are given, either orally or intravenously, and thus NAC could be an interferent present in patient serum samples.

Sulfhydryl groups exogenous to the patient's fluid also may contaminate the sample. Sulfhydryl-containing materials, such as 2-benzothiazolethiol, have been used in sulfur vulcanized rubber stoppers, such as those used on blood collection or transfer tubes. Sulfhydryl groups can leach out of the stopper into the fluid sample and interfere with the redox reaction of a clinical test.

Thus, the problem is that clinical analytical assays which use chemical schemes based on redox reactions can be adversely affected when high levels of free sulfhydryl groups are present in the test specimen. Any solution to this problem must take into account the fact that agents found to block such interference can also block reagents involved in the color-forming redox reaction of the assay itself.

EPA 0 309 882 A2 discloses a method and a reagent combination for eliminating reducing components from a test sample. In accordance with that disclosure, prior to the color reaction, the test sample is treated at neutral pH with one or more enzymatic oxidizing agents as well as with one or more sulfhydryl group-blocking substances, then the pH is adjusted to between 10 and 15 and the color reagent added.

The disadvantage of this method is that it requires one or more enzymes and that could be costly. It also requires two separate steps with different pH conditions, a requirement that is both time consuming and tedious. Further, the test is done in solution.

It would be desirable to have a dry clinical assay, requiring a single step, that can eliminate reducing interferents and accurately detect analytes in aqueous fluids.

The present invention eliminates high levels of free sulfhydryl groups from aqueous fluids to be assayed for the presence of analyte. The present invention introduces in one layer of a dry analytical element chemical groups which eliminate free sulfhydryl groups from the test specimen, thus preventing these interfering groups from reacting with reagents in other layers of the element. By using the element of the invention, reliable results can be obtained.

Accordingly, there is provided an analytical element for the determination of an analyte in an aqueous fluid comprising a porous spreading layer containing a reagent capable of binding free sulfhydryl groups present in the aqueous fluid.

Another embodiment of the invention provides a method of detecting an analyte in an aqueous fluid comprising contacting a sample of the aqueous fluid with a porous spreading layer superposed on a reagent layer in a dry analytical element, the spreading layer containing a material capable of complexing free sulfhydryl groups present in the sample, and the reagent layer containing reagents capable of reacting with the analyte to provide detectable spectrophotometric changes, thereby determining the presence of analyte.

Figure 1 is a graph showing the ability of n-acetylcysteine to bleach a formed color compound to a non-color form.

Figure 2 is a graph showing the ability of n-acetylcysteine to inhibit peroxidase activity.

Figure 3 is a graph showing the effect of gold chloride on n-acetylcysteine interference.

Figure 4 is a graph showing the effect of silver nitrate (A) and cupric sulfate (B) on n-acetylcysteine interference.

In order to eliminate from the test sample those components that have a reducing effect, we have incorporated sulfhydryl group-blocking agents into the spreading layer of a dry analytical element to which the specimen is applied. A sulfhydryl group-blocking agent is a reagent that reacts with one or more sulfhydryl groups such that the groups become nonreactive as reductants. One or more reagent layers contiguous to the spreading layer contain the reagents for detecting the analyte. It appears that the sulfhydryl interferents are complexed or eliminated in the spreading layer and are not available as reductants in the reagent layer. Unexpectedly, as shown in a comparative example below, the sulfhydryl-blocking agent must be incorporated into the spreading layer. They are not effective when incorporated into the reagent layer.

The following tests demonstrate that compounds which bear sulfhydryl groups, NAC for example, can interfere with redox-type assays by acting as reducing agents and preventing formation of color compounds (dyes) which signal the presence of analyte. The tests also show that sulfhydryl-blocking agents such as N-ethyl maleimide can eliminate the interference.

NAC is exemplary because it is reported to be an interferent in several redox-based assays that are dependent upon dye formation such as the Ektachem™ tests for acetaminophen, salicylate and creatinine assays. A marked reduction in color signal is observed when NAC is present in the sample. It appears that NAC interferes in a manner analogous to ascorbate interference. Like ascorbate, NAC appears to be a powerful reducing agent that interferes with color formation either by inhibiting the reagent enzyme in the assay cascade, by reducing the enzyme (for example horse radish peroxidase) so that no dye is produced, or by reducing the oxidized color product to a non-color form (that is, by "bleaching" the formed dye).

Sulfhydryl group-blocking agents are substances that will react with and bind sulfhydryl groups. Suitable blocking agents are known in the art, for example, iodoacetamide, iodoacetate, and N-ethylmaleimide. See, for example, EPA 0 309 882 A2 at pages 6 and 8. Particularly useful in dry analytical elements are maleimide, N-ethylmaleimide, iodoacetamide, silver nitrate, gold chloride or combinations thereof. Conjugates of these agents (such as maleimide on a polymer or bead) also may be used.

Analytes which can be detected using the element of the invention are acetaminophen, salicylate, creatinine, cholesterol, HDL cholesterol, triglycerides, glucose, and uric acid. This list includes also all tests in which enzymes produce hydrogen peroxide which is assayed by way of peroxidase-coupled redox chemistry steps.

Materials: Eastman Kodak Company, Rochester, NY, provided the inorganic salts and two unique quinones identified as KAN 908056 and KAN 090914, 4-aminoantipyrine (4-AAP), phenol, methione, cysteine, maleimide, iodoactamide, Ellman's Reagent, o-dianisidine, horseradish peroxidase, and 2{tris-(hydroxymethyl)aminomethane} (TRIS) buffer were purchased from Sigma Chemical Co., St. Louis, MO. Sarcosine oxidase (SOX) was from Toyo Jozo. All solutions were made with distilled, deionized water.

As used in the tests below, one unit of enzyme refers to that amount of enzyme required to generate 1 $\mu$M of product per minute under the conditions defined.

Effect of NAC on Peroxidase Activity

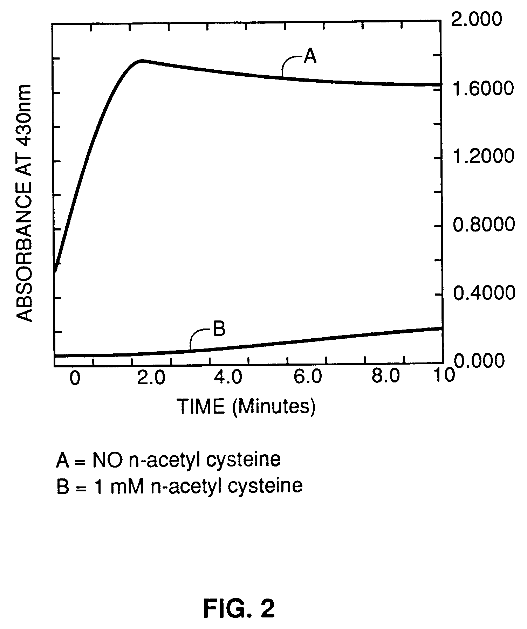

One mL reaction solution was prepared containing 100 mM TRIS-C1, pH 7.75, 0.88 mM $H_2O_2$, and 0.157 mM o-dianisidine. All reagents were combined in the assay cuvette and incubated at 37°C for 10 minutes after which time 0.59 Unit of horse radish peroxidase was added to the solution to initiate reaction. Reactions were monitored spectrophotometrically at 430 nm; millimolar extinction coefficient was 11.3. When 1 mM NAC was included in the reaction mixture, peroxidase activity, as monitored by dye formation, was significantly inhibited. Results are shown in Figure 2.

Effect of NAC on Sarcosine Oxidase (SOX) Activity

A standard SOX reaction solution can be prepared containing in 1 mL; 100 mM TRIS-C1, pH 7.75, 200 mM sarcosine, 1.5 mM 4-AAP, 0.02% (by weight) phenol, and 5.9 Units peroxidase. All reagents are combined in the assay cuvette and incubated at 37°C for 10 minutes after which time 1 Unit/mL SOX is added to the solution to initiate reaction. Reactions are observed spectrophotometrically at 505 nm; millimolar extinction coefficient is 17.4.

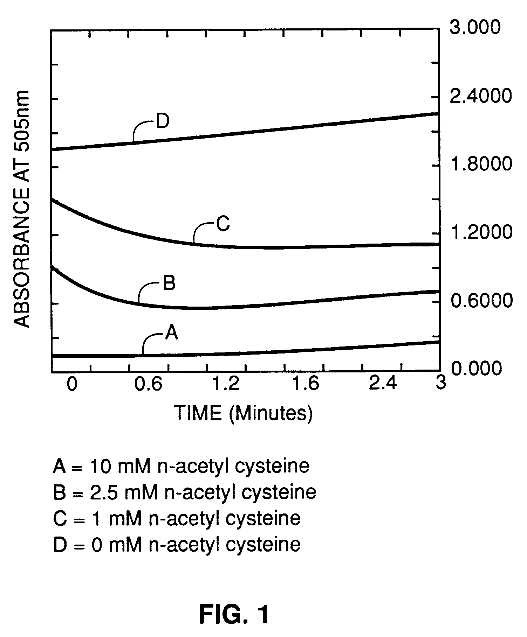

When determining the effect of NAC on SOX activity, the standard test described above is carried out except that to allow the reaction to go to completion before NAC is added, only 0.1 mM sarcosine is added. Thus, we ensured that all color formation was complete (the sarcosine being depleted). Indicator dye

4

formed in four cuvettes when SOX was added; absorbance endpoint was 1,800. NAC then was added to the cuvettes to the final concentration of 0, 1, 2.5 or 10 mM, and the changes in absorption monitored. Rapidly (less than 10 seconds) each cuvette containing MAC lost color, proportional to MAC concentration. Final absorbances were 1.880, 1.100, 0.600 and 0.150 for cuvette containing 0, 1, 2.5 or 10 mM NAC, respectively. Results are shown in Figure 1. These results demonstrate that MAC can affect the indicator dye signal in redox-type assays, even when the indicator dye is preformed.

Effect of Sulfhydryl-Blocking Reagents on NAC Interference

A variety of materials were selected as examples of reagents that might complex with (bind) the sulfhydryl group of NAC and thus block interference. Each material was screened in the following set of reaction mixtures:
a) standard SOX assay as described above (no NAC added)
b) a) plus 1 mM NAC
c) a) plus 1 mM NAC plus 1 mM of blocking reagent to be tested
d) a) plus 1 mM blocking reagent (no NAC)
Based on the results of this test, materials tested are listed in three groups shown in Table 1. One group contains materials not effective under the conditions tested; a second group appeared to minimize NAC interference, but adversely affected the assay. For example, the solution became gel-like or color formation was impaired. It is possible that this group of materials would be effective if they were incorporated apart from the detection chemistry, for example, either attached to beads or polymers or in a separate layer in a multilayer slide format.

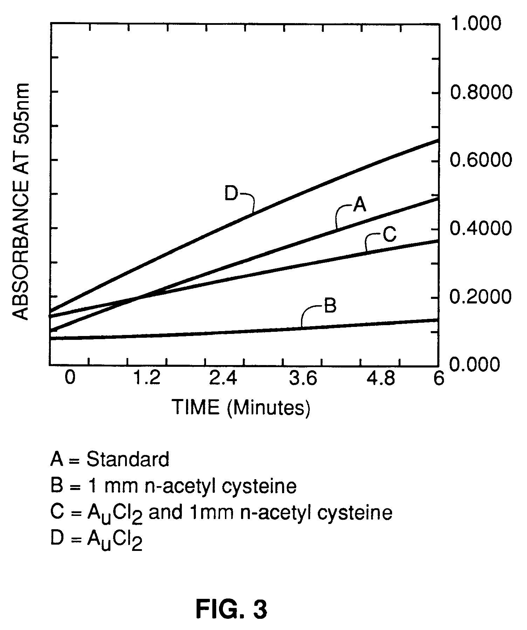

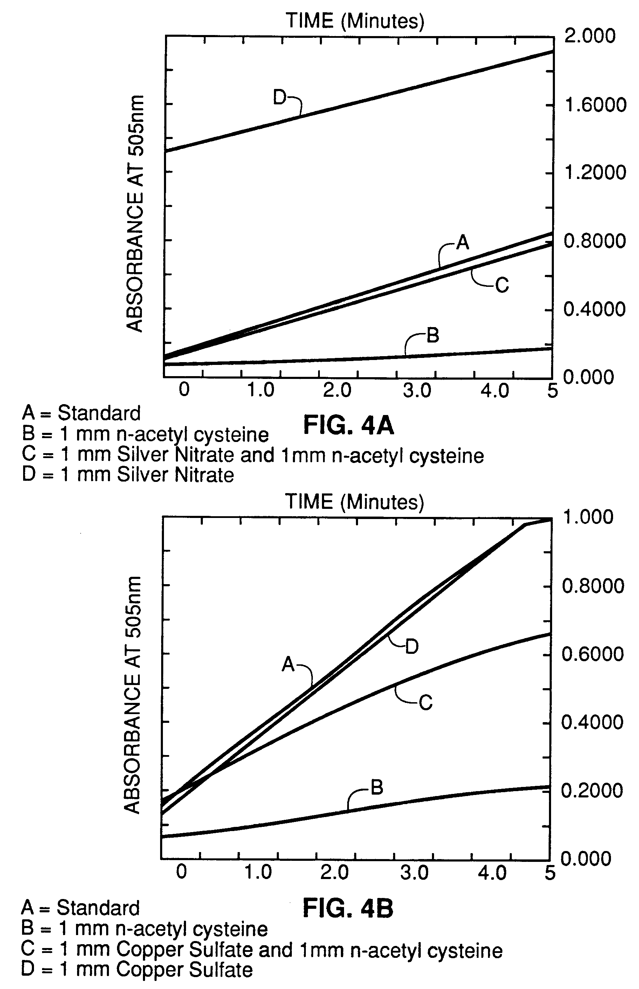

The third group contains materials that effectively eliminated NAC interference and had no effect on dye formation or enzyme rate. In each case, the blocking reagent alone (set d) had no effect on the detection system. When this group of blocking reagents and NAC were included in the reaction cuvette, SOX reaction rates improved markedly. Response from test sets using silver nitrate (A), and copper sulfate (B), are presented in Figure 4. Results with gold chloride are shown in Figure 3.

## TABLE 1
## Materials screened for effectiveness
## in removing NAC interference

Group 1. **Non-effective**
Zinc Chloride
Lead Nitrate
Nickel Sulfate
Cobalt Chloride
Ferric Chloride
4,4'-dinitropyridine
P-quinone
KAN 908056
KAN 090914

Group 2. **Effective but React with Dye**
Gold Chloride
5',5-dithio,bis,2-nitrobenzoil acid

Group 3. **Effective and Non-reactive with Dye**
Cupric Sulfate
N-ethyl-maleimide
Silver Nitrate
Silver Sulfate

Concentration Dependent Response

It appears that the ability of the blocking reagent to eliminate interference depends on the concentration of blocking agent relative to the amount of interferent present in the fluid. Tests carried out with a SOX assay as described above, with N-ethyl maleimide as blocking reagent and NAC as interferent, illustrate the concentration-dependent nature of the response. If there is more NAC than N-ethyl maleimide, NAC remains an interferent and no improvement in enzyme rate is observed. However, when an excess of N-ethyl maleimide was added (as blocking reagent), observed reaction rates were similar to those seen with the control (that is, no NAC added).

Compounds identified above in solution tests as useful sulfhydryl-blocking reagents were further tested for their effectiveness in a dry analytical element. Methods of preparation are disclosed in US-A-3,992,158, US-A-4,357,363, and US-A-4,258,001.

Example 1: Element for Salicylate Assay

In this example, a dry analytical element formulated for measuring salicylate was used. The lower reagent layers of the salicylate element (Table II) were overcoated with either the standard spreading layer (that is, without a blocking reagent) or a spreading layer which included one of the blocking reagents to be tested. A series of serum samples containing known levels of salicylate were spotted on the various test elements. Absorbance was measured at 540 nm after a 5 minute incubation at 37°C. From this series, a relationship between element absorbance and salicylate level could be made.

Serum samples containing 40 mg/dL of salicylate (Fluid A) and either 100 mg/dL of NAC (Fluid B) or a one hour exposure to a stopper containing 2-benzothiazolethiol (Fluid C) were then spotted on the elements and their predicted salicylate levels determined. Results are shown in Table III below.

## Table II
### Salicylate Element
### With and Without Blocking Agent

| Spreading Layer | Useful Range | Actual Range |
|---|---|---|
| AgNO$_3$ (not in standard element) | 0.1-5 g/m$^2$ | 0.4 g/m$^2$ |
| TiO$_2$ | 10-100 g/m$^2$ | 53 g/m$^2$ |
| Cellulose Acetate | 1-20 g/m$^2$ | 7.8 g/m$^2$ |
| Surfactant TX-405™* | .1-3 g/m$^2$ | 1.4 g/m$^2$ |
| Surfactant Brij 78* | .1-3 g/m$^2$ | 0.7 g/m$^2$ |
| Estane | .5-8 g/m$^2$ | 2.4 g/m$^2$ |
| Reagent Layer 1 | | |
| Gelatin | 1-20 g/m$^2$ | 6 g/m$^2$ |
| MBTH dye * | .1-2 g/m$^2$ | .4 g/m$^2$ |
| Tyrosinase | 100-100,000 U/m$^2$ | 960 U/m$^2$ |
| Reagent Layer 2 | | |
| Gelatin | 1-20 g/m$^2$ | 16 g/m$^2$ |
| TRIS buffer pH 7.8* | 1-12 g/m$^2$ | 3.3 g/m$^2$ |
| NADH * | .2-5 g/m$^2$ | 1 g/m$^2$ |
| Salicylate hydrogenase | 50-5,000 U/m$^2$ | 576 U/m$^2$ |

* MBTH: (3-methyl-2-benzothiazolinone hydrazone)

* NADH: (nicotinamide adenine dinucleotide)

* Zonyl FSN™: A family of octylphenoxy and polyethoxy
  TX102™     ethanol sold by Rohm & Haas Co.
  TX165™
  TX405™

* Brij 78:     polyoxyethylene alcohol

* TRIS: tris(hydroxymethyl)aminomethane}

Table III

| Blocking Agent Added to Standard Element | Salicylate Prediction of Fluid | | |
|---|---|---|---|
| | Fluid A | Fluid B | Fluid C |
| None | 40 mg/dL | 0.0 mg/dL | 15 mg/dL |
| 0.2 g/m$^2$ HAuC1$_4$ | 40 mg/dL | 40 mg/dL | 40 mg/dL |
| 0.34 g/m$^2$ AgNO$_3$ | 40 mg/dL | 40 mg/dL | 40 mg/dL |
| 0.18 g/m$^2$ | 40 mg/dL | 12 mg/dL | 42 mg/dL |
| Iodoacetamide 0.2 g/m$^2$ Fe(NO)$_3$ | 40 mg/dL | 0.0 mg/dL | 17 mg/dL |
| .5 g/m$^2$ Maleimide | 40 mg/dL | 40 mg/dL | 28 mg/dL |
| 0.5 g/m$^2$ Maleimide and 2 g/m$^2$ Iodoacetamide | 40 mg/dL | 40 mg/dL | 39 mg/dL |

7

Thus, for the salicylate element, $AgNO_3$, maleimide, maleimide with iodoacetamide, and $HAuCl_4$ were all shown to work well in reducing sulfhydryl interference.

Example 2: Placement of the Blocking Reagent

Placement of the blocking reagent in the proper layer of the dry element is essential for stability. In this example, we placed the blocking reagent (0.5 $g/m^2$ of maleimide) in the spreading layer of one element, a location that is relatively hydrophobic in nature, and in the reagent layer of another element. When placed in the reagent layer, the blocking agent quickly became ineffective. Results are shown in Table IV.

Table IV

| Fluid Description | Standard Element | Std w/ Maleimide in Spreading Layer | Std. w/ Maleimide in Reagent layer |
|---|---|---|---|
| Serum w/ Salicylate | 30mg/dL | 30mg/dL | 30mg/dL |
| As above w/ 100mg/dL N-acetyl cysteine | 0.0mg/dL | 30.1mg/dL | 0.3mg/dL |

Example 3: Salicylate Element With and Without Silver Nitrate

Standard elements (without blocking reagent) were spotted with solutions containing human serum spiked with salicylate in concentrations between 0 and 60 mg/dL. After a 5 minute incubation at 37°C the reflectance density was measured at 540 nm. Predicted salicylate concentration can be determined by fitting a response curve through the reflectance densities produced. Salicylate predictions made using known concentrations of salicylate in sera are shown in Table V.

Table V

| Salicylate Level | Density Produced | Salicylate Prediction |
|---|---|---|
| 0.0 mg/dL | .3114 | 0.3 mg/dL |
| 4 mg/dL | .5798 | 4.1 mg/dL |
| 13 mg/dL | .7077 | 14 mg/dL |
| 20 mg/dL | .7696 | 21 mg/dL |
| 30 mg/dL | .8391 | 31 mg/dL |
| 40 mg/dL | .9188 | 42 mg/dL |
| 52 mg/dL | .9748 | 52 mg/dL |
| 60 mg/dL | 1.0156 | 59 mg/dL |

Serum samples containing salicylate and sulfhydryl-containing interferents were then spotted on the standard element and on the element of the invention containing 0.4 $g/m^2$ of silver nitrate added to the spreading layer. Table VI shows the difference between the predicted values and the values actually obtained from two elements--one the standard element and one the element of the invention.

## Table VI

| Salicylate at 15 mg/dL | Standard element | Std w/ AgNO₃ |
|---|---|---|
| n-acetylcysteine at 102 mg/dL | -11.32 mg/dL bias | -0.01 mg/dL bias |
| 1 hour contact w/ rubber stoppers containing 2-benzothiazolethiol | -12.9 mg/dL bias | 0.3 mg/dL bias |

### Salicylate at 40 mg/dl

| | | |
|---|---|---|
| n-acetylcysteine at 102 mg/dL | -40 mg/dL bias | -1.2 mg/dL bias |
| 1 hour contact w/ rubber stoppers containing 2-benzothiazolethiol | -31.5 mg/dL bias | 0.3 mg/dL bias |

A comparison between the interference levels between the standard element for salicylate and the new element of the invention shows that sulfhydryl interference has been reduced to insignificance.

Example 4: Acetaminophen Element With and Without Maleimide

This example is similar to Example 3 except that the analyte is acetaminophen and the element used for testing is the one shown in Table VII below. The Table shows the standard element; the element of the invention is similar but it contains also 1 $g/m^2$ maleimide in the spreading layer.

Human serum spiked with acetaminophen varying in concentration between 1 and 294 mg/dL were spotted on the standard element and the element of the invention. After incubating for 5 minutes at 37°C, reflectance density was measured at 670 nm. Predictions of acetaminophen concentration were obtained as was done for salicylate concentration in Example 3. Differences between the predicted values and the values actually obtained from the two elements (standard and the invention) are shown in Table VIII.

A comparison of the interference levels between the standard element for acetaminophen and the element of the invention shows that the interference from NAC is almost eliminated by the invention.

9

## Table VII
### Standard Acetaminophen Element

<u>Coverage g/m$^{2*}$</u>

| Reagents/Spreading Layer | Useful Range | Actual Range |
|---|---|---|
| Barium Sulfate | 75-120 | 100 |
| Cellulose Acetate | 1-20 | 8 |
| Surfactant TX-100™ | 0.2-2 | 0.7 |
| Estane | .1-15 | 1.08 |
| **Subbing Layer** | | |
| Polyvinylpyrrolidone | 0.5-2.5 | 1.5 |
| **Reagent Layer 1** | | |
| Hardened Gelatin | 4.24 | 6 |
| Surfactant TX-165™ | 0.005-0.1 | 0.01 |
| Buffer | 2-10 | 2.4 |
| Ascorbic Acid Oxidase | 50,000- 300,000 U/m$^2$ | 250,000 U/m$^2$ |
| Arylacylamidase | 1,000- 50,000 u/m$^2$ | 5,000 U/m$^2$ |
| **Reagent Layer 2** | | |
| Unhardened Gelatin | 4.24 | 6 |
| Coupler THQSO$_3$H | 0.2-2 | 1.0 |
| Buffer | 2-10 | 4.8 |
| Surfactant TX-165™ | 0.005-0.1 | 0.01 |

*g/m$^2$ except for ascorbic acid oxidase and arylacylamidase which are IU/m$^2$

**Buffers:** tris(hydroxymethyl)aminomethane (TRIS); 2{[tris(hydroxymethyl)methyl)amino}-1-ethanesulfonic acid (TES); 4-(2-Hydroxyethyl)-1-piperazine-ethanesulfonic acid (HEPES); 3-[4-(2-Hydroxyethyl)-1-piperazinyl]propanesulfonic acid (EPPES); 2-Hydroxy-3-{N-[tris(hydroxymethyl)methyl]-amino}propanesulfonic acid (TAPSO); and other buffers with pH in the range of 6.5 to 8.5, preferably 7.5.
**Surfactants:** Surfactants such as TX-405™, TX-102™ and Zonyl FSN™ may also be used.
**THQSQ$_3$H Coupler:** (1-(3-sulfopropyl-1,2,3,4-tetrahydroquinoline)

| Acetaminophen at 1 mg/dL | Standard Element | std w/ 1 mg/m² maleimide |
|---|---|---|
| n-acetylcysteine at 100 mg/dL | -1.0 mg/dL bias | 0 mg/dL bias |
| Acetaminophen at 52 mg/dl | | |
| n-acetylcysteine at 100 mg/dL | -52 mg/dL bias | -.5 mg/dL bias |
| Acetaminophen at 202 mg/dL | -200 mg/dL bias | -11 mg/dL bias |
| n-acetylcysteine at 100 mg/dL | | |

**Claims**

1. An analytical element for the determination of an analyte in an aqueous fluid characterized in that the element comprises a porous spreading layer containing a reagent capable of binding free sulfhydryl groups present in the aqueous fluid.

2. The analytical element of claim 1 wherein the reagent capable of binding free sulfhydryl groups is selected from the group consisting of maleimide, N-ethyl maleimide, iodoacetamide, silver nitrate, gold chloride, and combinations thereof.

3. The analytical element of claim 1 wherein the analyte is acetaminophen, salicylate, creatinine, cholesterol, HDL cholesterol, triglycerides, glucose, or uric acid.

4. The analytical element of claim 1 further containing a reagent layer in fluid contact with the spreading layer.

5. The analytical element of claim 4 wherein the reagent layer contains aryl acylamidase, ascorbic acid oxidase, and a water soluble coupling agent and the analyte is acetaminophen.

6. The analytical element of claim 5 wherein the sulfhydryl group-binding reagent is maleimide.

7. The analytical element of claim 4 wherein the reagent layer contains tyrosinase, 3-methyl-2-benzothiazolinone hydrazone, nicotinamide adenine dinucleotide, and salicylate hydrogenase, and the analyte is salicylate.

8. The analytical element of claim 7 wherein the sulfhydryl group-binding reagent is silver nitrate.

9. An analytical element for the determination of salicylate comprising a support having thereon, in order from the support:
   a) at least one reagent layer containing tyrosinase, 3-methyl-2-benzothiazolinone hydrazone, nicotinamide adenine dinucleotide, and salicylate hydrogenase; and
   b) a porous spreading layer containing silver nitrate.

10. A method of detecting an analyte in an aqueous fluid comprising contacting a sample of the aqueous fluid with the spreading layer of the element defined in any of the preceding claims and determining the presence of the analyte by detectable spectrophotometric changes in the element.

11. A method of detecting an analyte in an aqueous fluid comprising contacting a sample of the aqueous fluid with a porous spreading layer superposed on a reagent layer in a dry analytical element, the spreading layer containing a material capable of complexing free sulfhydryl groups present in the sample, and the reagent layer containing reagents capable of reacting with the analyte to provide detectable spectrophotometric changes, thereby determining the presence of analyte.

12. The method of claim 10 or 11 wherein the analyte is selected from the group consisting of acetaminophen, salicylate, creatine kinase, cholesterol, HDL cholesterol, triglycerides, glucose, and uric acid.

13. The method of claim 10 or 11 wherein the sulfhydryl group-complexing material is maleimide, N-ethyl maleimide, iodoacetamide, silver nitrate, gold chloride, or combinations thereof.

**FIG. 1**

A = 10 mM n-acetyl cysteine
B = 2.5 mM n-acetyl cysteine
C = 1 mM n-acetyl cysteine
D = 0 mM n-acetyl cysteine

FIG. 2

A = NO n-acetyl cysteine
B = 1 mM n-acetyl cysteine

A = Standard
B = 1 mm n-acetyl cysteine
C = $A_uCl_2$ and 1mm n-acetyl cysteine
D = $A_uCl_2$

**FIG. 3**

**FIG. 4A**

A = Standard
B = 1 mm n-acetyl cysteine
C = 1 mm Silver Nitrate and 1mm n-acetyl cysteine
D = 1 mm Silver Nitrate

**FIG. 4B**

A = Standard
B = 1 mm n-acetyl cysteine
C = 1 mm Copper Sulfate and 1mm n-acetyl cysteine
D = 1 mm Copper Sulfate

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,D | EP - A - 0 309 882 (BOEHRINGER MANNHEIM GMBH) * Claims, especially claim 11 * | 11-13 | C 12 Q 1/00 G 01 N 33/52 C 12 Q 1/32 C 12 Q 1/50 C 12 Q 1/54 |
| A | EP - A - 0 409 345 (EASTMAN KODAK COMPANY) * Abstract; claims * | 1,3, 11,12 | C 12 Q 1/60 C 12 Q 1/61 C 12 Q 1/62 |
| A | EP - A - 0 239 990 (KONISHIROKU PHOTO INDUSTRY CO. LTD.) * Abstract; claims 1,20,21 * | 1-3, 11-13 | |
| A | PATENT ABSTRACTS OF JAPAN, unexamined application, C section, vol. 13, no. 562, December 13, 1989 THE PATENT OFFICE JAPANESE GOVERNMENT page 11 C 665 * No. 1-231 897 (FUJI PHOTO FILM CO. LTD.) | 1-4 | |
| A | US - A - 4 990 457 (TANAKA et al.) * Abstract; claims 1-3 * | 1 | TECHNICAL FIELDS SEARCHED (Int. Cl.5) C 12 Q G 01 N |
| A | EP - A - 0 123 443 (FUJI PHOTO FILM CO. LTD.) * Abstract; figs. * | 1,3, 11,12 | |
| D,A | US - A - 3 992 158 (PRZYBYLOWICZ et al.) * Abstract; claims, especially claims 33-36 * | 1,3, 11,12 | |
| A | US - A - 4 567 024 (KOYAMA et al.) * Abstract * | 1 | |

The present search report has been drawn up for all claims

| Place of search VIENNA | Date of completion of the search 23-09-1993 | Examiner SCHNASS |
|---|---|---|

EPO FORM 1503 03.82 (P0401)